# EUROPÄISCHE PATENTSCHRIFT

(11) **EP 0 628 563 B1**
(45) Veröffentlichungstag und Bekanntmachung des Hinweises auf die Patenterteilung: **03.03.1999**
(21) Anmeldenummer: 94108215.8
(22) Anmeldetag: 28.05.1994
(51) Int. Cl.: C07D 521/00, C07D 231/12

(54) **Verfahren zur Herstellung von N-substituierten Pyrazolen**
Process for the preparation of N-substituted pyrazoles
Procédé pour la préparation de pyrazoles N-substituées

(30) Priorität: 08.06.1993 DE 4318960; 08.02.1994 DE 4403815
(43) Veröffentlichungstag der Anmeldung: 14.12.1994
(73) Patentinhaber: BASF Aktiengesellschaft, 67063 Ludwigshafen (DE)
(72) Erfinder: Ebel, Klaus, Dr., D-67067 Ludwigshafen (DE); Schroeder, Juergen, Dr., D-67071 Ludwigshafen (DE); Groening, Carsten, Dr., D-68259 Mannheim (DE); Dockner, Toni, Dr., D-67149 Meckenheim (DE); Merkle, Hans Rupert, Dr., D-67061 Ludwigshafen (DE)

(56) Entgegenhaltungen:
- EP-A- 0 516 982
- FR-A- 1 603 793
- CHEMISTRY LETTERS, Nr.4, 1992, TOKYO JP Seiten 575 - 578 N. TANAKA ET AL. 'Transition Metal-Catalyzed N-Alkylation of NH Groups of Azoles with Alcohols'
- CHEMICAL ABSTRACTS, vol. 90, no. 3, 15. Januar 1979, Columbus, Ohio, US; abstract no. 22552s, Y. MOTOYAMA ET AL. 'N-Alkyl aromatic amines.' Seite 605 ;Spalte 1 ; & JP-A-53 090 227 (MITSUI PETROCHEMICAL INDUSTRIES, LTD.) 08 August 1978
- Liebigs Ann.Chem.598,186-193
- Houben-Weyl,Methoden der organischen Chemie,Bd.E8c,Seite 132
- J.Heterocycl.4,451 (1967)
- Ber.dtsch.chem.Ges.41,4010-4011 (1908)
- Liebigs Ann.Chem.593,202-205 (1955)
- Chem.Ber.85,823 (1952)
- J.Chem.Soc.99,2055 (1911)
- Liebigs Ann.Chem.343,304-305 (1923)
- Helv.Chim.Acta 11,952 (1928)
- Liebigs Ann.Chem.305,299 (1899)
- C.Elschenbroich und A.Salzer:Organometallics,2 Aufl.,1992,411-412
- Römpp Chemie Lextion,9.Aufl.,1990,2172-2173

## Beschreibung

Die vorliegende Erfindung betrifft ein Verfahren zur Herstellung von N-substituierten Pyrazolen durch Umsetzung von Pyrazolen mit Alkoholen bzw. Ethern in Gegenwart eines Heterogenkatalysators bei erhöhten Temperaturen.

Aus der FR-A 1 603 973 ist ein Verfahren zur Alkylierung von Imidazolen mit Alkoholen oder Ethern durch heterogen katalysierte Umsetzung mit Oxiden oder Phosphaten der Metalle der 2., 3. oder 4. Gruppe des Periodensystems bekannt. Imidazole und Pyrazole unterscheiden sich jedoch gravierend hinsichtlich ihrer Reaktivität.

Aus der EP-A-454 307 ist ein Verfahren zur N-Alkylierung von Pyrazolen bekannt, bei dem N-unsubstituierte Pyrazole mit starken Basen wie z.B. Natriummethylat oder Alkalimetallen wie z.B. Natrium in das entsprechende Salz überführt und anschließend mit einem Alkylhalogenid oder Dialkylsulfat alkyliert werden. Nachteilig bei diesem Verfahren sind die teuren Ausgangsstoffe und der hohe Salzanfall.

Aus Chemistry Letters, 575 bis 578 (1992) ist ein Verfahren zur N-Alkylierung von Pyrazolen bekannt, bei dem N-unsubstituierte Pyrazole mit Alkoholen in Gegenwart katalytischer Mengen von Ruthenium-, Rhodium- oder Iridium-Trialkylphosphit-Komplexen umgesetzt werden.

Nachteilig bei diesem Verfahren ist der hohe Preis für die Katalysatoren.

Der vorliegenden Erfindung lag daher die Aufgabe zugrunde, den zuvor genannten Nachteilen abzuhelfen.

Demgemäß wurde ein neues und verbessertes Verfahren zur Herstellung von N-substituierten Pyrazolen der allgemeinen Formel I 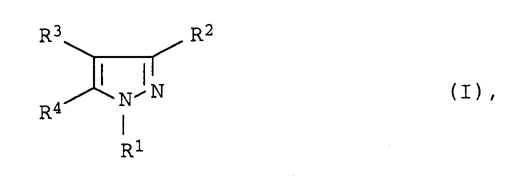 in der
- R¹: C₁- bis C₁₂-Alkyl oder C₇- bis C₂₀-Phenylalkyl und
- R²,R³,R⁴: unabhängig voneinander Wasserstoff, C₁- bis C₁₂-Alkyl, Phenyl, C₇- bis C₂₀-Alkylphenyl oder C₇- bis C₂₀-Phenylalkyl
bedeuten, durch Umsetzung von Pyrazolen der allgemeinen Formel II 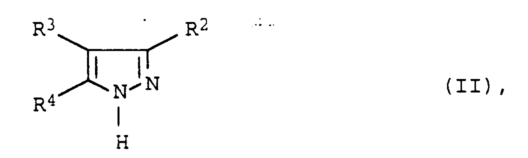 in der R², R³ und R⁴ die obengenannten Bedeutungen haben, mit Verbindungen der allgemeinen Formel III

R¹ - O - R⁵ (III),

in der R¹ die obengenannten Bedeutungen hat und
- R⁵: Wasserstoff oder R¹ bedeuten,
in Gegenwart eines Katalysators bei Temperaturen von 200 bis 550°C und Drücken von 0,001 bis 50 bar, gefunden, welches dadurch gekennzeichnet ist, daß man als Katalysator einen Heterogenkatalysator einsetzt.

Das erfindungsgemäße Verfahren läßt sich wie folgt durchführen:

Die Umsetzung kann durch in Kontaktbringen von einem Pyrazol II und einer Verbindung III in Gegenwart eines Heterogenkatalysators bei Temperaturen von 200 bis 550°C, bevorzugt 250 bis 450°C, besonders bevorzugt 280 bis 400°C und einem Druck von 0,001 bis 50 bar, bevorzugt 0,01 bis 5 bar, besonders bevorzugt 0,1 und 1,5 bar, in der Regel bei Normaldruck (Atmosphärendruck) und gegebenenfalls einem Inertgas wie Stickstoff und Argon durchgeführt werden. Temperatur und Druckbedingungen sollten so gewählt werden, daß die Reaktion in der bevorzugten Gasphase stattfindet. Als Katalysatoren eignen sich Heterogenkatalysatoren, besonders solche mit aciden Zentren wie Aluminiumoxid, siliciumdioxid, Titandioxid und/oder Zirkondioxid, gegebenenfalls mit 1 bis 30, insbesondere 5 bis 15 Gew.-% Phosphorsäure dotiert, bevorzugt sind gamma-Aluminiumoxid und Siliciumdioxid. Die Phosphorsäure kann ganz oder teilweise als Phosphorpentoxid, Orthophosphorsäure, Pyrophosphorsäure oder Polyphosphorsäure, z.B. von 72 bis 88 Gew.-% Phosphorpentoxid, vorliegen und wird hier als Phosphorsäure berechnet, unabhängig von der tatsächlichen Konstitution der Phosphorsäure oder Phosphorsäureanhydrids.

Das Molverhältnis von Verbindung III zum Pyrazol II beträgt in der Regel von 0,01 : 1 bis 1,1 : 1, bevorzugt 0,1 : 1 bis 1 : 1, besonders bevorzugt 0,8 : 1 bis 0,95 : 1.

Die Reaktion kann so durchgeführt werden, daß man die auf Reaktionstemperatur erhitzten Ausgangsstoffe II und III über den auf Reaktionstemperatur erhitzten Katalysator in einem Festbettreaktor leitet. Das den Reaktionsraum verlassende Gemisch kann kondensiert und fraktioniert destilliert werden.

Im Vergleich zu den bekannten Verfahren liefert das erfindungsgemäße Verfahren auf einfacheren und wirtschaftlicherem Wege N-substituierte Pyrazole.

Die Substituenten R¹, R², R³, R⁴ und R⁵ in den Verbindungen I, II und III haben folgende Bedeutungen:
R¹,R²,R³,R⁴,R⁵
   unabhängig voneinander
   - C₁- bis C₁₂-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl,
   - Phenyl,
   - C₇- bis C₂₀-Alkylphenyl, bevorzugt C₇- bis C₁₂-Alkylphenyl wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
   - C₇- bis C₂₀-Phenylalkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenylbutyl und 4-Phenyl-butyl, besonders bevorzugt Benzyl, 1-Phenethyl und 2-Phenethyl,
R²,R³,R⁴,R⁵
   - unabhängig voneinander
   - Wasserstoff.

Als Ausgangsverbindung II eignen sich besonders Pyrazole wie Pyrazol, 3-Methylpyrazol, 4-Methylpyrazol, 3,4-Dimethylpyrazol, 3,5-Dimethylpyrazol, 3-Ethylpyrazol, 4-Ethylpyrazol, 3-Phenyl-pyrazol, 4-Phenylpyrazol, 3,4-Diphenylpyrazol, 3,5-Diphenylpyrazol und 3-Methyl-4-phenylpyrazol.

Als Ausgangsverbindung III eignen sich besonders Alkohole wie Methanol, Ethanol, n-Propanol, Isopropanol und n-Butanol sowie Ether wie Dimethylether, Diethylether, Di-n-propylether und Diisopropylether.

Die nach dem erfindungsgemäßen Verfahren herstellbaren N-substituierten Pyrazole I sind wertvolle Ausgangsstoffe bei der Herstellung von Farbstoffen, Pharmazeutika und Pflanzenschutzmitteln.

### Beispiele

### Beispiel 1

Stündlich werden 65,6 g einer Mischung aus 7,5 Gew.-% 3,5-Diphenylpyrazol, 10,9 Gew.-% Methanol und 81,6 Gew.-% Toluol verdampft und mit 10 Nl/h Stickstoff in Sumpffahrweise durch einen auf 380°C erhitzten Festbettreaktor geleitet. Der Festbettreaktor ist mit 50 ml eines Katalysators aus 80 Gew.-% gamma-Aluminiumoxid und 20 Gew.-% Siliciumdioxid gefüllt. Die Reaktionsgase werden anschließend kondensiert. Man erhält stündlich 55,7 g organische Phase mit 9,0 Gew.-% 1-Methyl-3,5-diphenylpyrazol (96 % der Theorie bezogen auf eingesetztes 3,5-Diphenylpyrazol).

### Beispiel 2

Stündlich werden 65,6 g einer Mischung aus 7,5 Gew.-% 3,5-Diphenylpyrazol, 10,9 Gew.-% Methanol und 81,6 Gew.-% Toluol verdampft und mit 10 Nl/h Stickstoff in Sumpffahrweise durch einen auf 410°C erhitzten Festbettreaktor geleitet. Der Festbettreaktor ist mit 50 ml eines Katalysators aus 80 Gew.-% gamma-Aluminiumoxid und 20 Gew.-% Siliciumdioxid gefüllt. Die Reaktionsgase werden anschließend kondensiert. Man erhält stündlich 57,3 g organische Phase mit 8,6 Gew.-% 1-Methyl-3,5-diphenylpyrazol (94 % der Theorie bezogen auf eingesetztes 3,5-Diphenylpyrazol).

### Beispiel 3

Stündlich werden 87,0 g einer Mischung aus 6,0 Gew.-% 3,5-Diphenylpyrazol, 12,5 Gew.-% Ethanol und 81,5 Gew.-% Toluol verdampft und mit 10 Nl/h Stickstoff in Sumpffahrweise durch einen auf 410°C erhitzten Festbettreaktor geleitet. Der Festbettreaktor ist mit 50 ml eines Katalysators aus 80 Gew.-% gamma-Aluminiumoxid und 20 Gew.-% Siliciumdioxid gefüllt. Die Reaktionsgase werden anschließend kondensiert. Man erhält stündlich 74,4 g organische Phase mit 1,4 Gew.-% 1-Methyl-3,5-diphenylpyrazol und 5,9 Gew.-% 1-Ethyl-3,5-diphenylpyrazol (94 % der Theorie bezogen auf eingesetztes 3,5-Diphenylpyrazol).

### Beispiel 4

Stündlich werden 87,0 g einer Mischung aus 6,0 Gew.-% 3,5-Diphenylpyrazol, 12,5 Gew.-% Methanol und 81,5 Gew.-% Toluol verdampft und mit 10 Nl/h Stickstoff in Sumpffahrweise durch einen auf 450°C erhitzten Festbettreaktor geleitet. Der Festbettreaktor ist mit 50 ml eines Katalysators aus 80 Gew.-% gamma-Aluminiumoxid und 20 Gew.-% Siliciumdioxid gefüllt. Die Reaktionsgase werden anschließend kondensiert. Man erhält stündlich 72,9 g organische Phase mit 2,3 Gew.-% 1-Methyl-3,5-diphenylpyrazol und 5,0 Gew.-% 1-Ethyl-3,5-diphenylpyrazol (92 % der Theorie bezogen auf eingesetztes 3,5-Diphenylpyrazol).

### Beispiel 5

Stündlich werden 25,5 g einer Mischung aus 12,2 Gew.-% 3-Phenylpyrazol, 27,2 Gew.-% Methanol und 60,6 Gew.-% Toluol verdampft und mit 10 Nl/h Stickstoff in Sumpffahrweise durch einen auf 410°C erhitzten Festbettreaktor geleitet. Der Festbettreaktor ist mit 50 ml eines Katalysators aus 80 Gew.-% gamma-Aluminiumoxid und 20 Gew.-% Siliciumdioxid gefüllt. Die Reaktionsgase werden anschließend kondensiert. Man erhält stündlich 19,0 g organische Phase mit 16,9 Gew.-% 1-Methyl-3-phenylpyrazol/1-Methyl-5-phenylpyrazol (95 % der Theorie bezogen auf eingesetztes 3-Phenylpyrazol).

### Beispiel 6

Stündlich werden 22,5 g einer Mischung aus 58,5 Gew.-% Methanol und 41,5 Gew.-% Pyrazol verdampft und mit 10 Nl/h Stickstoff in Sumpffahrweise durch einen auf 300°C erhitzten Festbettreaktor geleitet. Der Festbettreaktor ist mit 100 ml eines Katalysators aus 95 Gew.-% gamma-Aluminiumoxid und 5 Gew.-% Phosphorsäure gefüllt. Die Reaktionsgase werden anschließend kondensiert. Man erhält stündlich 17,0 g Kondensat mit 62,2 Gew.-% 1-Methylpyrazol (99 % der Theorie bezogen auf eingesetztes Pyrazol).

### Beispiel 7

Stündlich werden 22,5 g einer Mischung aus 58,5 Gew.-% Methanol und 41,6 Gew.-% Pyrazol verdampft und mit 10 Nl/h Stickstoff in Sumpffahrweise durch einen auf 450°C erhitzten Festbettreaktor geleitet. Der Festbettreaktor ist mit 100 ml eines Katalysators aus 95 Gew.-% gamma-Aluminiumoxid und 5 Gew.-% Phosphorsäure gefüllt. Die Reaktionsgase werden anschließend kondensiert. Man erhält stündlich 18,4 g Kondensat mit 41,8 Gew.-% 1-Methylpyrazol (68 % der Theorie, bezogen auf eingesetztes Pyrazol) und 11,7 Gew.-% 1,4-Dimethylpyrazol (16 % der Theorie, bezogen auf eingesetztes Pyrazol).

### Beispiel 8

Stündlich werden 21,5 g einer Mischung aus 66,1 Gew.-% Methanol und 33,9 Gew.-% 4-Methylpyrazol verdampft und mit 10 Nl/h Stickstoff in Sumpffahrweise durch einen auf 400°C erhitzten Festbettreaktor geleitet. Der Festbettreaktor ist mit 100 ml eines Katalysators aus 90 Gew.-% Siliciumdioxid und 10 Gew.-% gamma-Aluminiumoxid gefüllt. Die Reaktionsgase werden anschließend kondensiert. Man erhält stündlich 15,5 g Kondensat mit 52,3 Gew.-% 1,4-Dimethylpyrazol (95 % der Theorie, bezogen auf eingesetztes 4-Methylpyrazol).

### Beispiel 9

Stündlich werden 111,3 g einer Mischung aus 53,9 Gew.-% Methanol und 46,1 Gew.-% 4-Methylpyrazol verdampft und mit 10 Nl/h Stickstoff in Sumpffahrweise durch einen auf 400°C erhitzten Festbettreaktor geleitet. Der Festbettreaktor ist mit 100 ml eines Katalysators aus 90 Gew.-% gamma-Aluminiumoxid und 10 Gew.-% Siliciumdioxid gefüllt. Die Reaktionsgase werden anschließend kondensiert. Man erhält stündlich 91,7 g Kondensat mit 3,2 Gew.-% 4-Methylpyrazol und 57,0 Gew.-% 1,4-Dimethylpyrazol (87 % der Theorie, bezogen auf eingesetztes 4-Methylpyrazol).

### Beispiel 10

Stündlich werden 43,8 g einer Mischung aus 67,0 Gew.-% Ethanol und 33,0 Gew.-% Pyrazol verdampft und mit 10 Nl/h Stickstoff in Sumpffahrweise durch einen auf 400°C erhitzten Festbettreaktor geleitet. Der Festbettreaktor ist mit 100 ml eines Katalysators aus 90 Gew.-% gamma-Aluminiumoxid und 10 Gew.-% Siliciumdioxid gefüllt. Die Reaktionsgase werden anschließend kondensiert. Man erhält stündlich 31,7 g Kondensat mit 0,2 Gew.-% Pyrazol und 62,2 Gew.-% 1-Ethylpylpyrazol (97 % der Theorie, bezogen auf eingesetztes Pyrazol).

### Beispiel 11

Stündlich werden 66,4 g einer Mischung aus 53,9 Gew.-% Methanol und 46,1 Gew.-% Methylpyrazol verdampft und mit 10 Nl/h Stickstoff in Sumpffahrweise durch einen auf 400°C erhitzten Festbettreaktor geleitet. Der Festbettreaktor ist mit 100 ml eines Katalysators aus 90 Gew.-% gamma-Aluminiumoxid und 10 Gew.-% Siliciumdioxid gefüllt. Die Reaktionsgase werden anschließend kondensiert. Man erhält stündlich 53,4 g Kondensat mit 1,0 Gew.-% 3-Methylpyrazol und 56,7 Gew.-% 1,3-Dimethylpylpyrazol (84 % der Theorie, bezogen auf eingesetztes 3-Methylpyrazol).

### Beispiel 12

Stündlich werden 21,3 g einer Mischung aus 68,7 Gew.-% Isopropanol und 31,3 Gew.-% Methylpyrazol verdampft und mit 10 Nl/h Stickstoff in Sumpffahrweise durch einen auf 300°C erhitzten Festbettreaktor geleitet. Der Festbettreaktor ist mit 100 ml eines Katalysators aus 90 Gew.-% gamma-Aluminiumoxid und 10 Gew.-% Siliciumdioxid gefüllt. Die Reaktionsgase werden anschließend kondensiert. Man erhält stündlich 8,6 g Kondensat mit 20,2 Gew.-% 3-Methylpyrazol und 60,8 Gew.-% 1-Isopropyl-3-methylpyrazol/1-Isopropyl-5-methylpyrazol (67 % der Theorie, bezogen auf eingesetztes 3-Methylpyrazol).

### Beispiel 13

Stündlich werden 39,0 g einer Mischung aus 73,0 Gew.-% Diethylether und 27,0 Gew.-% Methylpyrazol verdampft und mit 10 Nl/h Stickstoff in Sumpffahrweise durch einen auf 400°C erhitzten Festbettreaktor geleitet. Der Festbettreaktor ist mit 100 ml eines Katalysators aus 90 Gew.-% gamma-Aluminiumoxid und 10 Gew.-% Siliciumdioxid gefüllt. Die Reaktionsgase werden anschließend kondensiert. Man erhält stündlich 20,3 g Kondensat mit 3,3 Gew.-% 3-Methylpyrazol und 58,1 Gew.-% 1-Ethyl-3-methylpyrazol/1-Ethyl-5-methylpyrazol (96 % der Theorie, bezogen auf eingesetztes 3-Methylpyrazol).

## Patentansprüche

1. Verfahren zur Herstellung von N-substituierten Pyrazolen der allgemeinen Formel I 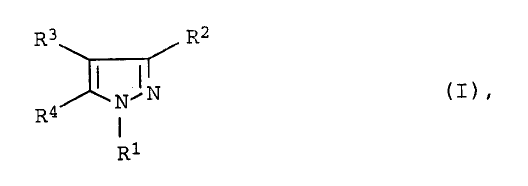 in der
R¹ C₁- bis C₁₂-Alkyl oder C₇- bis C₂₀-Phenylalkyl und
R²,R³,R⁴ unabhängig voneinander Wasserstoff, C₁- bis C₁₂-Alkyl, Phenyl, C₇- bis C₂₀-Alkylphenyl oder C₇-bis C₂₀-Phenylalkyl
bedeuten, durch Umsetzung von Pyrazolen der allgemeinen Formel II 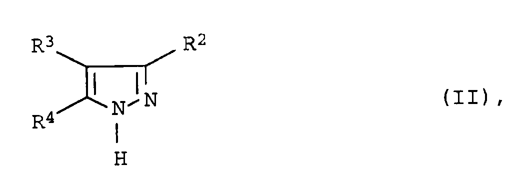 in der R², R³ und R⁴ die obengenannten Bedeutungen haben, mit Verbindungen der allgemeinen Formel III
R¹ - O - R⁵ (III),
in der R¹ die obengenannten Bedeutungen hat und
R⁵ Wasserstoff oder R¹ bedeuten,
in Gegenwart eines Katalysators bei Temperaturen von 200 bis 550°C und Drücken von 0,001 bis 50 bar, dadurch gekennzeichnet, daß man als Katalysator einen Heterogenkatalysator ausgewählt aus der Gruppe von Aluminiumoxid, Siliciumdioxid, Titandioxid und/oder Zirkondioxid, gegebenenfalls mit Phosphorsäure dotiert, einsetzt.

2. Verfahren zur Herstellung von N-substituierten Pyrazolen der Formel I nach Anspruch 1, dadurch gekennzeichnet, daß man als Katalysator einen Heterogenkatalysator mit aciden Zentren einsetzt.

3. Verfahren zur Herstellung von N-substituierten Pyrazolen der Formel I nach Anspruch 1, dadurch gekennzeichnet, daß man das Pyrazol II zur Verbindung III im Molverhältnis von 0,01 : 1 bis 1,1 : 1 einsetzt.

4. Verfahren zur Herstellung von N-substituierten Pyrazolen der Formel I nach Anspruch 1, dadurch gekennzeichnet, daß man das Pyrazol II zur Verbindung III im Molverhältnis von 0,1 : 1 bis 1 : 1 einsetzt.

## Claims

1. A process for preparing N-substituted pyrazoles of the formula I 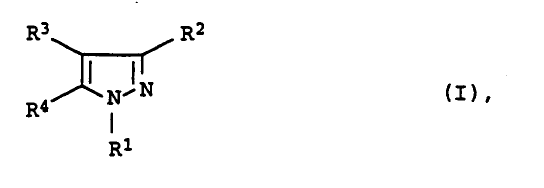 in which
R¹ is C₁- to C₁₂-alkyl or C₇- to C₂₀-phenylalkyl and
R²,R³,R⁴ independently of one another are hydrogen, C₁-to C₁₂-alkyl, phenyl, C₇- to C₂₀-alkylphenyl or C₇- to C₂₀-phenylalkyl
by reaction of pyrazoles of the formula II 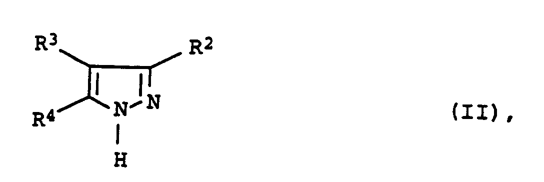 in which R², R³ and R⁴ are as defined above with compounds of the formula III
R¹ - O - R⁵ (III),
in which R¹ is as defined above and
R⁵ is hydrogen or R¹,
in the presence of a catalyst at temperatures of from 200 to 550°C and pressures of from 0.001 to 50 bar, which comprises using as catalyst a heterogeneous catalyst selected from the group consisting of aluminum oxide, silicon dioxide, titanium dioxide and/or zirconium dioxide, if appropriate doped with phosphoric acid.

2. A process for preparing N-substituted pyrazoles of the formula I as claimed in claim 1, wherein the catalyst used is a heterogeneous catalyst having acidic centers.

3. A process for preparing N-substituted pyrazoles of the formula I as claimed in claim 1, wherein the molar ratio of the pyrazole II to the compound III is from 0.01 : 1 to 1.1 : 1.

4. A process for preparing N-substituted pyrazoles of the formula I as claimed in claim 1, wherein the molar ratio of the pyrazole II to the compound III is from 0.1 : 1 to 1 : 1.

## Revendications

1. Procédé pour la préparation de pyrazoles N-substitués de formule générale I 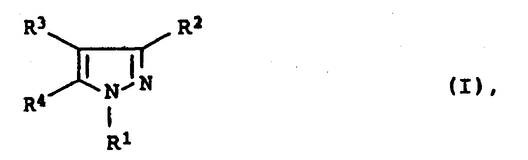 dans laquelle
R¹ représente un groupe alkyle en C₁-C₁₂ ou phénylalkyle en C₇-C₂₀ et
R², R³, R⁴ représentent, indépendamment les uns des autres, un atome d'hydrogène ou un groupe alkyle en C₁-C₁₂, phényle, alkylphényle en C₇-C₂₀ ou phénylalkyle en C₇-C₂₀,
par la réaction de pyrazoles de formule générale II 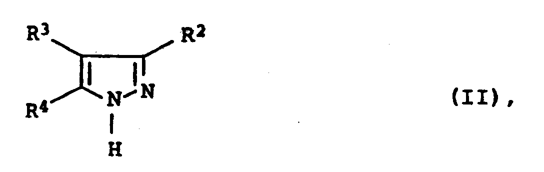 dans laquelle R², R³ et R⁴ ont les significations données ci-dessus, avec des composés de formule générale III
R¹ - O - R⁵ (III),
dans laquelle R¹ a les significations données ci-dessus et
R⁵ représente un atome d'hydrogène ou R¹,
en présence d'un catalyseur, à des températures de 200 à 550°C et sous des pressions de 0,001 à 50 bar , caractérisé en ce que l'on utilise comme catalyseur un catalyseur hétérogène choisi dans l'ensemble constitué par l'oxyde d'aluminium, le dioxyde de silicium, le dioxyde de titane et/ou le dioxyde de zirconium, éventuellement dopé à l'acide phosphorique.

2. Procédé pour la préparation de pyrazoles N-substitués de formule I selon la revendication 1, caractérisé en ce que l'on utilise comme catalyseur un catalyseur hétérogène à centres acides.

3. Procédé pour la préparation de pyrazoles N-substitués de formule I selon la revendication 1, caractérisé en ce que l'on utilise le pyrazole II, relativement au composé III, en un rapport molaire allant de 0,01:1 à 1,1:1.

4. Procédé pour la préparation de pyrazoles N-substitués de formule I selon la revendication 1, caractérisé en ce que l'on utilise le pyrazole II, relativement au composé III, en un rapport molaire allant de 0,1:1 à 1:1.
